(19) Europäisches Patentamt
European Patent Office
Office européen des brevets

(11) **EP 3 387 020 B1**

(12) **FASCICULE DE BREVET EUROPEEN**

(45) Date de publication et mention de la délivrance du brevet:
**31.01.2024 Bulletin 2024/05**

(21) Numéro de dépôt: **16825824.2**

(22) Date de dépôt: **12.12.2016**

(51) Classification Internationale des Brevets (IPC):
*C08B 30/18* $^{(2006.01)}$  *C08L 3/02* $^{(2006.01)}$
*A61K 47/36* $^{(2006.01)}$  *A23L 29/212* $^{(2016.01)}$

(52) Classification Coopérative des Brevets (CPC):
**C08B 30/18; A23L 29/212; A23L 29/35; C08L 3/02;**
A61K 47/26

(86) Numéro de dépôt international:
**PCT/FR2016/053326**

(87) Numéro de publication internationale:
**WO 2017/098191 (15.06.2017 Gazette 2017/24)**

(54) **HYDROLYSAT D'AMIDON DE BASSE VISCOSITE PRESENTANT UN COMPORTEMENT A LA RETROGRADATION AMELIORE**

NIEDRIGVISKOSES STÄRKEHYDROLYSAT MIT VERBESSERTEM RETROGRADATIONSVERHALTEN

LOW-VISCOSITY STARCH HYDROLYSATE WITH IMPROVED RETROGRADATION BEHAVIOUR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.12.2015 FR 1562123**

(43) Date de publication de la demande:
**17.10.2018 Bulletin 2018/42**

(73) Titulaire: **Roquette Frères**
**62136 Lestrem (FR)**

(72) Inventeurs:
• **IBERT, Mathias**
**59930 La Chapelle D'armentieres (FR)**
• **LECOCQ, Aline**
**59420 MOUVAUX (FR)**
• **LANOS, Pierre**
**59480 La Bassee (FR)**

(74) Mandataire: **Plasseraud IP**
**66, rue de la Chaussée d'Antin**
**75440 Paris Cedex 09 (FR)**

(56) Documents cités:
EP-A1- 0 905 256      WO-A1-2004/064540
WO-A1-2011/017093      WO-A2-01/96537
FR-A1- 2 203 877      FR-A1- 2 762 616
GB-A- 2 001 075

Remarques:
Le document complet y compris le(s) table(s) de référence et le(s) listage(s) de séquence sont téléchargeables sur le site internet de l'OEB

**Description**

**Domaine de l'invention**

L'invention a pour objet un nouvel procédé de fabrication de hydrolysat d'amidon. Il est également divulgué l'utilisation de cet hydrolysat dans les industries alimentaires et pharmaceutiques.

L'invention a plus précisément pour but de fournir un hydrolysat d'amidon présentant un bon comportement à la rétrogradation, tout en conservant un goût et une odeur plus neutres et peu sucrés, ainsi qu'une texture en bouche améliorée.

**Art antérieur**

Les hydrolysats d'amidon sont généralement regroupés sous deux grandes familles : les maltodextrines lorsqu'ils présentent un faible DE, généralement inférieur à 20, et les sirops de glucose lorsqu'ils présentent un DE de 20 ou plus.

Ces hydrolysats sont produits sous forme de sirops à partir d'amidon en suspension aqueuse, cet amidon étant éclaté par chauffage et hydrolysé en présence d'acide, d'enzyme ou un mélange d'acide et d'enzyme, pour transformer l'amidon en des carbohydrates de plus bas poids moléculaire. On obtient à l'issue de ce procédé un sirop, qui peut éventuellement être atomisé.

La composition de l'hydrolysat (ou encore profil glucidique) dépend de l'amidon de départ utilisé mais également du procédé d'hydrolyse utilisé. Par exemple, la conversion de l'amidon est réalisée par une première étape d'hydrolyse, couramment appelée « liquéfaction ».

On peut arrêter l'hydrolyse après cette étape afin d'obtenir des maltodextrines, dont le DE est facilement réglé par l'Homme du métier en faisant varier les conditions opératoires (durée, température, choix et quantités d'enzymes et/ou de pH, ...). Par rapport aux sirops de glucose, ces maltodextrines sont faiblement sucrées.

Au contraire, il est possible de laisser l'hydrolyse se poursuivre afin d'obtenir des sirops de glucose, qui présentent généralement un goût plus sucré. On réalise généralement ces sirops de glucose par une seconde étape d'hydrolyse, couramment appelée « saccharification ». Cette étape se fait généralement à plus basse température que l'étape de liquéfaction et dans des conditions opératoires différentes, par exemple en ajoutant une ou plusieurs enzymes.

En cas d'utilisation d'enzymes dans le procédé, leur sélection est évidemment essentielle sur le profil glucidique de l'hydrolysat obtenu.

Parmi les enzymes pouvant être utilisées dans des procédés de fabrication de sirops de glucose figurent les enzymes alpha-amylase commercialisées relativement récemment par la société Verenium sous les marques Veretase® et Fuelzyme®-LF. La fonction enzymatique de ces enzymes alpha-amylases est d'hydrolyser particulièrement les saccharides de haut moléculaire ; ceci permet notamment de réduire très rapidement la viscosité de l'hydrolysat d'amidon.

Dans la suite de la description, pour des raisons de simplicité, ces enzymes alpha-amylases hydrolysant particulièrement les saccharides de haut moléculaire sont regroupées sous le terme « Enzyme V ».

Les Enzymes V commercialisées par la société Verenium sous les marques Veretase® et Fuelzyme®-LF sont des enzymes choisies parmi les polypeptides encodés par une séquence nucléotidique telle que représentée en SEQ ID N°1 ou une séquence nucléotidique présentant un pourcentage d'identité par rapport à la séquence SEQ ID N°1 au moins égal à 80%, préférentiellement 85%, préférentiellement 90%, plus préférentiellement 98%, encore plus préférentiellement 99%.

La séquence SEQ ID N°1 représente une séquence nucléotidique codant pour une alpha amylase. Cette séquence est disponible en ligne sous le numéro d'accession GenBank AF504065.1.

Ces enzymes V sont également décrites dans les brevets US 7,273,740, US 7,666,633 et US 7,659,102. Ces documents décrivent également des procédés de fabrication de sirops très riches en glucose (dont la teneur est supérieure à 95% en glucose), ces procédés comprenant une étape de liquéfaction utilisant une Enzyme V ainsi qu'une étape de saccharification utilisant cette enzyme conjointement avec une enzyme additionnelle de type glucoamylase.

Egalement, parmi les documents décrivant la fabrication d'hydrolysats utilisant une Enzyme V, on peut citer la demande WO2009/137839, qui décrit essentiellement des hydrolysats qui sont des sirops de glucose. Ces hydrolysats présentent un DE allant de 20 à 52 de faible viscosité comprenant une teneur en sucres inférieure à 25%. Ces sirops sont de faible viscosité car ils comprennent une faible quantité de polysaccharides de DP supérieur ou égal à 15, conjointement avec une forte quantité d'oligosaccharides de DP allant de 3 à 14. Parmi les nombreuses variantes de fabrication de ces hydrolysats figure le mode de réalisation de l'exemple 18 ; celui-ci décrit un sirop de glucose fabriqué par un procédé comprenant une étape de liquéfaction d'amidon en présence d'une amylase de type Enzyme V suivie d'une étape de saccharification utilisant cette enzyme conjointement avec des enzymes additionnelles qui sont une isoamylase et une pullulanase. Les sirops obtenus présentent un DE de 29 ou 30 et comprennent une quantité nulle de polysaccharides de DP supérieur ou égal à 15.

Le document WO2013/116175 décrit quant à lui de la même manière un procédé de fabrication d'un sirop de glucose comprenant une étape d'hydrolyse de l'amidon utilisant une Enzyme V pour obtenir, avant l'étape de filtration, un hydrolysat ayant un profil glucidique tel que les quantités de DP1-2 aillent de 10 à 25%, de DP3-11 aillent de 70 à 90% et les quantités de DP12 et plus soient inférieures à 15%. Ces sirops présentent également les mêmes avantages : une teneur en sucres réduite par rapport à un sirop de glucose de DE supérieur, tout en présentant une viscosité similaire.

Le mélange d'alpha-amylases décrit dans la demande WO2011/017093 porte sur un mélange d'une Enzyme V et d'une enzyme *Bacillus Licheniformis,* ces enzymes étant dans des quantités relatives allant de 0,5 à 5 *Liquefon Units* (LUs) de *Bacillus Licheniformis* pour 5 Modified Wohlgemuth Units (MWUs) Enzyme V. Ce mélange vise à résoudre le problème suivant : lorsque l'on réalise la fabrication d'un sirop très riche en glucose en réalisant une étape de liquéfaction utilisant comme alpha-amylase exclusivement une Enzyme V, le sirop très riche en glucose obtenu à l'issue de la saccharification présente des résultats au test d'iode positif, ce qui indique que l'hydrolyse de l'amidon est incomplète. Ce document décrit la fabrication de sirops de glucose de haut DE fabriqué par un procédé comprenant une étape de liquéfaction d'amidon en présence du mélange d'alpha amylase de type Enzyme V et *Bacillus Licheniformis* suivie d'une étape de saccharification utilisant ce mélange d'enzyme conjointement avec un mélange d'enzyme glucoamylase et pullulanase additionnel (Optimax® 4060 VHP).

Les hydrolysats d'amidon sont dans la plupart des cas mis en solution avant d'être utilisés, par exemple dans la fabrication d'aliments. En ce qui concerne les hydrolysats d'amidon obtenus par des alpha-amylases classiques, par exemple l'enzyme Liquozyme® Supra (Novozymes®), un des problèmes récurrents est qu'ils peuvent ne pas être stables lorsqu'ils sont mis en solution. On parle d'un phénomène de rétrogradation. La solution d'hydrolysat devient alors inhomogène, ce qui rend complexe son utilisation ultérieure et demande généralement de resolubiliser l'hydrolysat rétrogradé avant de pouvoir utiliser la solution d'hydrolysat. Ce phénomène est généralement observé au bout de quelques jours. Toutefois, ce phénomène de rétrogradation des hydrolysats est plus rapidement observé pour des hydrolysats présentant un DE inférieur à 10, particulièrement lorsque le DE est inférieur à 5.

De ce qui précède, on constate que, si des procédés de fabrication de sirops de glucose utilisant une Enzyme V lors de l'étape de liquéfaction ont déjà été décrits, on constate qu'aucun de ces documents ne s'est intéressé au problème de la rétrogradation des hydrolysats d'amidon. Or, contrairement aux hydrolysats obtenus par les alpha-amylases classiques, la Demanderesse a constaté que, de manière surprenante, les hydrolysats ayant un DE de 30 ou moins et qui sont fabriqués par un procédé comprenant une étape de liquéfaction d'amidon utilisant une Enzyme V, rétrogradent rapidement. Ce phénomène de rétrogradation peut être observé dans le cas des hydrolysats de DE inférieur ou égal à 30 et il l'est tout particulièrement dans le cas des maltodextrines de DE inférieur à 20.

Dans le cadre de ses recherches, la Demanderesse est parvenue à fabriquer un nouvel hydrolysat d'amidon présentant un bon comportement à la rétrogradation, tout en conservant une viscosité plus basse que des hydrolysats de DE équivalent fabriqués par des alpha-amylases classiques (autres qu'une Enzyme V). Cet hydrolysat est caractérisé par un profil glucidique tout à fait particulier.

**Résumé de l'invention**

Il est divulgué un hydrolysat d'amidon présentant un Dextrose Equivalent DE allant de 5 à 30 et dans lequel le DE, la teneur en poids sec de saccharides de degré de polymérisation allant de 10 à 20 (DP 10-20) et la teneur en poids sec de saccharides de degré de polymérisation de 50 ou plus (DP 50+) sont telles qu'ils répondent aux inéquations suivantes :

$$-0,83 \times DE + 25 \leqslant \%DP50+ \leqslant -1,07 \times DE + 40 \ ;$$

$$-0,83 \times DE + 27,5 \leqslant \%DP\ 10\text{-}20 \leqslant -1,25 \times DE + 55 \ .$$

L'hydrolysat présente de manière surprenante un très bon comportement à la rétrogradation.

Il présente en outre l'avantage, lorsqu'il est mis en solution, de présenter une basse viscosité.

Ceci lui permet d'être facilement manipulé et utilisé comme matière première dans des produits alimentaires et pharmaceutiques. Il présente en outre d'autres avantages tels qu'un goût et une odeur neutres, peu sucrés du fait de leur DE mais plus que les hydrolysats classiques, et une très bonne texture en bouche.

La Demanderesse est parvenue à fabriquer l'hydrolysat en mettant en oeuvre un procédé particulier.

L'invention porte sur un procédé de fabrication d'un hydrolysat d'amidon comprenant :

a) une étape de mise en contact d'une solution aqueuse d'amidon ou de matériau amylacé avec au moins une alpha-amylase choisie parmi les Enzymes V ;

b) suivie d'une étape d'hydrolyse de ladite solution aqueuse ;

c) suivie d'une étape d'inhibition de l'Enzyme V pour former une solution d'hydrolysat intermédiaire présentant un DE égal à $DE_c$ ;

d) suivie d'une étape de mise en contact de la solution d'hydrolysat intermédiaire obtenue à l'étape c) avec au moins une alpha-amylase additionnelle, différente de l'Enzyme V ;

e) suivie d'une seconde étape d'hydrolyse pour former un hydrolysat pendant un temps suffisant pour qu'il présente un Dextrose Equivalent $DE_e$ allant de 5 à 30 ;

f) suivie d'une étape de récupération de l'hydrolysat formé à l'étape e) ;

caractérisé en ce que l'Enzyme V est une enzyme choisie parmi les :

- les polypeptides encodés par une séquence nucléotidique telle que représentée en SEQ ID N°1 ou une séquence nucléotidique présentant un pourcentage d'identité par rapport à la séquence SEQ ID N°1 au moins égal à 80%, préférentiellement 85%, préférentiellement 90%, plus préférentiellement 98%, encore plus préférentiellement 99%, ou
- les polypeptides comprenant le fragment enzymatiquement actif de la séquence codée par la séquence SEQ ID N°1.

Selon une variante de l'invention, l'Enzyme V est une enzyme présentant un résultat au test A inférieur à 10%, le test A consistant à :

- réaliser une d'étape d'hydrolyse à un pH de 5,3 d'un amidon de maïs natif avec, comme seule enzyme, l'enzyme à tester jusqu'à ce qu'un hydrolysat d'amidon de DE égal à 20 soit obtenu,
- déterminer la teneur en DP50+ dudit hydrolysat, le résultat au test A étant égal à cette teneur en DP50+.

Au contraire des enzymes citées dans les documents FR 2 203 877 A1, GB 2 001 075 A, WO 01/96537 A2, EP 0 905 256 A1, WO 2004/064540 A1 et FR 2 762 616 A1, l'enzyme V de l'invention permet ainsi de réduire très fortement la teneur en saccharides en DP50+ en comparaison avec les enzymes classiques. Parmi les enzymes classiques, on peut citer l'enzyme Termamyl 120 L.

Comme montré dans les exemples, ce procédé spécifique, utilisant cette Enzyme V particulière, a permis d'obtenir le profil glucidique particulier de l'hydrolysat de l'invention.

L'hydrolysat présente notamment, en comparaison avec un hydrolysat de même DE et fabriqué à partir d'une enzyme classique de type Termamyl, un goût plus sucré, ceci alors que les teneurs en sucres (c'est-à-dire en DP1 et DP2) sont très similaires.

L'invention va maintenant être décrite en détail dans la suite de la description.

## Description détaillée des figures

La Figure 1 représente l'évolution de la teneur en poids sec de DP1-2 en fonction du DE d'hydrolysats selon l'invention et comparatifs.

La Figure 2 représente l'évolution de la teneur en poids sec de DP3-6 en fonction du DE d'hydrolysats selon l'invention et comparatifs.

La Figure 3 représente l'évolution de la teneur en poids sec de DP7-9 en fonction du DE d'hydrolysats selon l'invention et comparatifs.

La Figure 4 représente l'évolution de la teneur en poids sec de DP10-20 en fonction du DE d'hydrolysats selon l'invention et comparatifs.

La Figure 5 représente l'évolution de la teneur en poids sec de DP21-30 en fonction du DE d'hydrolysats selon l'invention et comparatifs.

La Figure 6 représente l'évolution de la teneur en poids sec de DP31-40 en fonction du DE d'hydrolysats selon l'invention et comparatifs.

La Figure 7 représente l'évolution de la teneur en poids sec de DP41-50 en fonction du DE d'hydrolysats selon l'invention et comparatifs.

La Figure 8 représente l'évolution de la teneur en poids sec de DP50+ en fonction du DE d'hydrolysats selon l'invention et comparatifs.

## Description détaillée de l'invention

Il est divulgué un hydrolysat d'amidon particulier que l'on va décrire en détail par la suite. L'hydrolysat présente un Dextrose Equivalent (DE) allant de 5 à 30. Le DE définit le degré d'hydrolyse de l'amidon. Plus particulièrement, le DE, exprimé en pourcents est déterminé par la mesure du pouvoir réducteur de l'hydrolysat d'amidon en comparaison avec le pouvoir réducteur du glucose. Le DE peut être mesuré en utilisant la méthode NF EN ISO 5377:1981.

Le profil glucidique de l'hydrolysat présente une distribution particulière de saccharides.

Le terme « saccharides » inclut selon l'invention, tous les types de saccharides, c'est-à-dire les monosaccharides, les disaccharides, les oligosaccharides et les polysaccharides.

Pour définir les saccharides et cette distribution, on utilise le terme DP X dans lequel X représente le nombre d'unité glucose dans le saccharide. Egalement, les saccharides de degré de polymérisation comprenant un nombre d'unités glucose allant de X à Y (X et Y inclus) sont regroupés sous le terme saccharides de DP X-Y. En outre, les saccharides de degré de polymérisation comprenant un nombre d'unités glucose de plus de X (X non inclus) sont regroupés sous le terme saccharides de DP X+.

Par « saccharides de haut poids moléculaire », on entend les saccharides DP50+.

La composition comprend, selon son DE, des teneurs spécifiques en saccharides de DP 10-20 et en saccharides de DP 50+. Les teneurs en saccharides sont exprimées en poids sec, c'est-à-dire qu'elles expriment la quantité en poids sec de ces saccharides par rapport à la quantité en poids sec de la totalité des saccharides dans l'hydrolysat.

Ces teneurs peuvent être déterminées par chromatographie en phase liquide à haute performance sur résine échangeuse d'ions (HPLC pour High Performance Liquid Chromatography) et par chromatographie d'exclusion stérique (SEC pour Size Exclusion Chromatography). L'appareil HPLC peut être équipé d'une colonne de résine styrène divinyl benzène échangeuse d'ions mise sous forme d'argent, par exemple une colonne de type Bio-Rad HPX 42A, et d'un détecteur à indice de réfraction. L'appareil SEC peut être équipé de colonnes polymère polyhydroxymethylmethacrylate, par exemple de colonnes OHpak SB-802 HQ, OHpak SB-803 HQ et OHpak SB-805 HQ placées dans cet ordre. L'appareil SEC peut être calibré à l'aide de standards Pullulans. Des protocoles de préparation des échantillons et de mesure sont présentés dans la partie Exemples.

Pour les saccharides en DP X-Y dont la teneur va jusqu'à 19, la teneur est déterminée directement par HPLC. En ce qui concerne les saccharides en DP X-Y dont le DP est de 20 ou plus (DP 20-30, DP 31-40, DP 41-50 et DP50+), leurs teneurs sont déterminées de la manière suivante :

- la teneur en DP 20+ est déterminée par HPLC selon la méthode ci-dessus ;
- le ratio (DP X-Y/DP20+) est déterminé par SEC selon la méthode ci-dessus ;
- la teneur en DP X-Y est déterminée par la formule suivante :

$$\text{DP X-Y} = \text{DP 20+} \times (\text{ratio DPX-Y/DP20+}).$$

L'hydrolysat d'amidon présente un Dextrose Equivalent DE allant de 5 à 30 et dans lequel le DE, la teneur en poids sec de saccharides de degré de polymérisation allant de 10 à 20 (DP 10-20) et la teneur en poids sec de saccharides de degré de polymérisation de 50 ou plus (DP 50+) sont telles qu'ils répondent aux inéquations suivantes :

- $$-0,83 \times \text{DE} + 25 \leqslant \%\text{DP 50+} \leqslant -1,07 \times \text{DE} + 40 \ ;$$

- $$-0,83 \times \text{DE} + 27,5 \leqslant \%\text{DP 10-20} \leqslant -1,25 \times \text{DE} + 55 \ .$$

De préférence, le DE de l'hydrolysat de l'invention est inférieur à 20, tout préférentiellement va de 10 à 19,5.

Dans ces gammes de DE, le comportement à la rétrogradation est encore plus amélioré par rapport à un hydrolysat de même DE fabriqué par un procédé comprenant une étape de liquéfaction d'amidon utilisant une Enzyme V.

L'hydrolysat d'amidon présente :

- un DE allant de 24 à 30 ;

- une teneur en poids sec de DP 10-20 allant de 10 à 18%;
- une teneur en poids sec de DP 50+ allant de 3 à 13%.

L'hydrolysat d'amidon présente avantageusement :

- une teneur en poids sec de DP 1-2 allant de 6 à 14% ;
- et/ou une teneur en poids sec de DP 3-6 allant de 35 à 55% ;
- et/ou une teneur en poids sec de DP 7-9 allant de 5 à 25% ;
- et/ou une teneur en poids sec de DP 21-30 allant de 2 à 8% ;
- et/ou une teneur en poids sec de DP 31-40 allant de 1 à 4% ;
- et/ou une teneur en poids sec de DP 41-50 allant de 1 à 4% .

L'hydrolysat d'amidon présente :

- un DE allant de 20 à 24 ;
- une teneur en poids sec de DP 10-20 allant de 10 à 20% ;
- une teneur en poids sec de DP 50+ allant de 6 à 18%.

L'hydrolysat d'amidon présente avantageusement :

- une teneur en poids sec de DP 1-2 allant de 4 à 10% ;
- et/ou une teneur en poids sec de DP 3-6 allant de 25 à 50% ;
- et/ou une teneur en poids sec de DP 7-9 allant de 13 à 30% ;
- et/ou une teneur en poids sec de DP 21-30 allant de 2 à 8% ;
- et/ou une teneur en poids sec de DP 31-40 allant de 2 à 5% ;
- et/ou une teneur en poids sec de DP 41-50 allant de 1 à 4%.

L'hydrolysat d'amidon présente :

- un DE allant de 17 à 20 ;

- une teneur en poids sec de DP 10-20 allant de 12 à 25% ;

- une teneur en poids sec de DP 50+ allant de 10 à 19%.

L'hydrolysat d'amidon présente avantageusement :

- une teneur en poids sec de DP 1-2 allant de 2 à 7% ;

- et/ou une teneur en poids sec de DP 3-6 allant de 15 à 35% ;

- et/ou une teneur en poids sec de DP 7-9 allant de 17% à 26% ;

- et/ou une teneur en poids sec de DP 21-30 allant de 3 à 10% ;

- et/ou une teneur en poids sec de DP 31-40 allant de 2 à 6% ;

- et/ou une teneur en poids sec de DP 41-50 allant de 2 à 4%.

L'hydrolysat d'amidon présente :

- un DE allant de 15 à 17 ;

- une teneur en poids sec de DP 10-20 allant de 16 à 27% ;

- une teneur en poids sec de DP 50+ allant de 13 à 22%.

L'hydrolysat d'amidon présente avantageusement :

- une teneur en poids sec de DP 1-2 allant de 2 à 6% ;

- et/ou une teneur en poids sec de DP 3-6 allant de 16 à 25% ;

- et/ou une teneur en poids sec de DP 7-9 allant de 17 à 24% ;

- et/ou une teneur en poids sec de DP 21-30 allant de 5 à 10% ;

- et/ou une teneur en poids sec de DP 31-40 allant de 3 à 6% ;

- et/ou une teneur en poids sec de DP 41-50 allant de 2 à 4%.

L'hydrolysat d'amidon présente :

- un DE allant de 13 à 15 ;

- une teneur en poids sec de DP 10-20 allant de 17 à 30% ;

- une teneur en poids sec de DP 50+ allant de 17 à 25%.

L'hydrolysat d'amidon présente avantageusement :

- une teneur en poids sec de DP 1-2 allant de 1 à 5% ;

- et/ou une teneur en poids sec de DP 3-6 allant de 13 à 23% ;

- et/ou une teneur en poids sec de DP 7-9 allant de 15 à 23% ;

- et/ou une teneur en poids sec de DP 21-30 allant de 6 à 12% ;

- et/ou une teneur en poids sec de DP 31-40 allant de 3 à 7% ;

- et/ou une teneur en poids sec de DP 41-50 allant de 2 à 4%.

L'hydrolysat d'amidon présente :

- un DE allant de 10 à 13 ;
- une teneur en poids sec de DP 10-20 allant de 20 à 32% ;
- une teneur en poids sec de DP 50+ allant de 18 à 28%.

L'hydrolysat d'amidon présente avantageusement :

- une teneur en poids sec de DP 1-2 allant de 0 à 4% ;
- et/ou une teneur en poids sec de DP 3-6 allant de 10 à 20% ;
- et/ou une teneur en poids sec de DP 7-9 allant de 15 à 22% ;
- et/ou une teneur en poids sec de DP 21-30 allant de 5 à 12% ;
- et/ou une teneur en poids sec de DP 31-40 allant de 4 à 8% ;
- et/ou une teneur en poids sec de DP 41-50 allant de 2 à 4%.

Les variantes 2 à 7 détaillées ci-dessus ne peuvent être combinées entre elles. *A contrario,* l'ensemble de ces variantes peuvent être combinées avec la première variante décrite ci-dessus.
Les variantes avantageuses et préférées décrites ci-après sont également combinables avec les variantes 1 à 7.
Généralement, la teneur en poids sec de DP11+ est supérieure à 15%, voire supérieure à 20%, par exemple supérieure à 25%.
De préférence, la teneur en poids sec de DP1-2 va de 1 à 15%. De préférence, la teneur en poids sec de DP3-6 va de 10 à 60%. De préférence, la teneur en poids sec de DP7-9 va de 5 à 30%. De préférence, la teneur en poids sec de DP21-30 va de 2 à 15%. De préférence, la teneur en poids sec de DP31-40 va de 1 à 6%. De préférence, la teneur en poids sec de DP41-50 va de 1 à 4%.

7

L'hydrolysat d'amidon peut être un hydrolysat d'amidon provenant de toutes origines botaniques, extrait des organes et tissus de réserve des végétaux supérieurs. Cet amidon peut être un amidon riche en amylose ou, inversement, riche en amylopectine (*waxy*). De préférence, la teneur sèche en amylose de l'amidon va de 0 à 85% et la teneur sèche en amylopectine va de 15 à 100%. Selon l'invention, le terme « amidon » regroupe les amidons et les fécules. L'amidon peut être choisi parmi les amidons de céréales telles que le blé, le maïs, l'orge, le triticale, le sorgo ou le riz, les tubercules tels que la pomme de terre ou le manioc, ou les légumineuses telles que le pois et le soja, et des mélanges de tels amidons. De préférence, l'hydrolysat selon l'invention est un hydrolysat d'amidon de blé, de maïs, de maïs *waxy,* de pois ou un hydrolysat de fécule de pomme de terre, tout préférentiellement de blé, de maïs, de maïs *waxy.*

L'hydrolysat peut notamment être utilisé dans les industries alimentaires et pharmaceutiques, notamment pour la fabrication d'aliments pour bébé, de boissons pour sportifs, de biscuits, de crèmes glacées, de sauces, de soupes, de boissons en poudre, de glaçages, de supports d'arômes, d'édulcorants. L'hydrolysat peut être utilisé en diététique infantile, en diététique clinique ou en diététique sportive. L'hydrolysat peut également être utilisé pour des fermentations diverses, notamment en boulangerie, en brasserie ou en charcuterie. L'hydrolysat peut également servir à la fabrication d'un mélange comprenant l'hydrolysat d'amidon de l'invention, notamment un mélange d'hydrolysat selon l'invention avec un hydrolysat additionnel, cet hydrolysat additionnel pouvant être choisi parmi les sirop riches en glucose et/ou en maltose.

L'hydrolysat comme il apparaît à la lumière des exemples, peut être obtenu par le procédé particulier de l'invention, dont les différents paramètres sont décrits en détail ci-après ; l'Homme du métier saura aisément faire varier ces paramètres afin d'obtenir ces hydrolysats selon l'invention.

L'invention porte sur un procédé de fabrication d'un hydrolysat d'amidon comprenant :

a) une étape de mise en contact d'une solution aqueuse d'amidon avec au moins une alpha-amylase choisie parmi les Enzymes V ;
b) suivie d'une étape d'hydrolyse de ladite solution aqueuse ;
c) suivie d'une étape d'inhibition de l'Enzyme V pour former une solution d'hydrolysat intermédiaire présentant un DE égal à $DE_c$ ;
d) suivie d'une étape de mise en contact de la solution d'hydrolysat intermédiaire obtenue à l'étape c) avec au moins une alpha-amylase additionnelle, différente de l'Enzyme V ;
e) suivie d'une seconde étape d'hydrolyse pour former un hydrolysat pendant un temps suffisant pour qu'il présente un Dextrose Equivalent $DE_e$ allant de 5 à 30 ;
f) suivie d'une étape de récupération de l'hydrolysat formé à l'étape e) ;

l'Enzyme V étant une enzyme présentant un résultat au test A inférieur à 10%, le test A consistant à :

• réaliser une d'étape d'hydrolyse à un pH de 5,3 d'un amidon de maïs natif avec, comme seule enzyme, l'enzyme à tester jusqu'à ce qu'un hydrolysat d'amidon de DE égal à 20 soit obtenu,
• déterminer la teneur en DP50+ dudit hydrolysat, le résultat au test A étant égal à cette teneur en DP50+.

Dans la première étape du procédé, on réalise une étape de mise en contact d'une solution aqueuse d'amidon ou de matériau amylacé avec au moins une alpha-amylase choisie parmi les Enzymes V.

L'amidon utilisé pour la fabrication de la solution aqueuse d'amidon de l'étape a) peut être de l'amidon natif, c'est-à-dire l'amidon sous forme granulaire obtenu par extraction des organes et tissus de réserve des végétaux supérieurs précédemment cités. L'amidon peut également être un amidon ayant subi une étape de modification. Cette étape de modification peut être une étape de prégélatinisation, de gélatinisation, d'oxydation ou d'hydrolyse acide. De préférence, l'amidon utilisé pour la fabrication de la solution aqueuse d'amidon est de l'amidon natif.

La solution aqueuse d'amidon peut comprendre une quantité d'amidon, exprimé en masse sèche d'amidon, allant de 10 à 50%, de préférence allant de 25 à 40%, par exemple de 30 à 35%. Un granule d'amidon natif comprend de l'eau liée. Le taux d'humidité de l'amidon natif, dans les conditions standard, varie selon la nature botanique de l'amidon. L'amidon natif comprend une quantité intrinsèque d'eau et le taux d'humidité dans cet amidon est généralement compris entre 10 et 20%. A titre d'exemple, un amidon de maïs natif présente une humidité dans les conditions standard d'environ 13%, tandis qu'une fécule de pomme de terre native présente une humidité d'environ 18%. La masse sèche de l'amidon peut être calculée selon la norme ISO 1666:1996.

Dans la solution aqueuse d'amidon est introduite, lors de l'étape a), au moins une alpha-amylase choisie parmi les Enzymes V.

Les Enzymes V hydrolysent particulièrement les saccharides de haut moléculaire. Ainsi, une Enzyme V est une enzyme présentant notamment un résultat au test A inférieur à 10%, voire inférieur à 5%.

L'étape d'hydrolyse du test A peut être réalisée à 95°C, éventuellement après une étape de cuisson à la vapeur de 5 minutes à 106°C. Pour réaliser l'étape d'hydrolyse du test A, on peut par exemple se référer aux conditions décrites au protocole 1 de la partie Exemples de la présente Demande.

L'Enzyme V de l'invention est une enzyme choisie parmi

- les polypeptides encodés par une séquence nucléotidique telle que représentée en SEQ ID N°1 ou une séquence nucléotidique présentant un pourcentage d'identité par rapport à la séquence SEQ ID N°1 au moins égal à 80%, préférentiellement 85%, préférentiellement 90%, plus préférentiellement 98%, encore plus préférentiellement 99%, ou
- les polypeptides comprenant le fragment enzymatiquement actif de la séquence codée par la séquence SEQ ID N°1.

Par "pourcentage d'identité" entre deux séquences d'acides nucléiques ou au sens de la présente invention, on entend désigner un pourcentage de nucléotides entre les deux séquences à comparer, obtenu après le meilleur alignement, ce pourcentage étant purement statistique et les différences entre les deux séquences étant réparties au hasard et sur toute leur longueur. On entend désigner par "meilleur alignement" ou "alignement optimal", l'alignement pour lequel le pourcentage d'identité déterminé comme ci-après est le plus élevé. Les comparaisons de séquences entre deux séquences d'acides nucléiques sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981), au moyen de l'algorithme d'homologie locale de Neddleman et Wunsch (1970), au moyen de la méthode de recherche de similarité de Pearson et Lipman (1988), au moyen de logiciels informatiques utilisant ces algorithmes (GAP, BESTFIT, BLAST P, BLAST N, FASTA et TFASTA dans le Wisconsin Genetics Software Package, Genetics Computer Group, 575 Science Dr., Madison, WI). Afin d'obtenir l'alignement optimal, on utilise de préférence le programme BLAST, avec la matrice BLOSUM 62.

Le pourcentage d'identité entre deux séquences d'acides nucléiques est déterminé en comparant ces deux séquences alignées de manière optimale, la séquence d'acides nucléiques à comparer pouvant comprendre des additions ou des délétions par rapport à la séquence de référence pour un alignement optimal entre ces deux séquences. Le pourcentage d'identité est calculé en déterminant le nombre de positions identiques pour lesquelles le nucléotide est identique entre les deux séquences, en divisant ce nombre de positions identiques par le nombre total de positions comparées et en multipliant le résultat obtenu par 100 pour obtenir le pourcentage d'identité entre ces deux séquences.

Lors de l'étape a), l'Enzyme V est mise en contact avec la solution d'amidon ou de matériau amylacé dans des quantités permettant d'obtenir le DE de l'hydrolysat intermédiaire lors de l'étape b). Cette quantité peut varier très largement et est directement liée aux conditions utilisées lors de cette étape b), notamment la durée, la température, et/ou le pH de la suspension aqueuse d'amidon. L'Enzyme V est généralement introduite sous forme de solutions commerciales d'enzymes. Par exemple, on peut l'introduire sous la forme d'une solution d'enzyme VERETASE® commercialisée par la société VERENIUM® ; la solution d'enzyme VERETASE peut être introduite dans des quantités massiques, exprimées par rapport à la masse sèche d'amidon, allant de 0,01 à 0,5%, de préférence de 0,03 à 0,13%. Une autre manière d'exprimer la quantité d'Enzyme V est de l'exprimer en quantité d'activité enzymatique de type « Modified Wolgemuth Unit » (MWU), bien connue de l'Homme du métier. Une unité MWU est la quantité d'enzyme permettant d'hydrolyser un milligramme d'amidon solubilisé en une dextrine spécifique au test MWU, en trente minutes et dans les conditions standard d'utilisation de l'enzyme. Par gramme d'amidon sec, cette quantité peut être comprise dans la gamme allant de 10 à 1000 MWU, par exemple de 50 à 500 MWU. A titre d'exemple, 1 g d'une solution d'enzyme VERETASE® comprend au moins 120000 MWU.

Selon une variante du procédé, une alpha-amylase supplémentaire, différente de l'Enzyme V est également mise en contact avec la suspension aqueuse d'amidon lors de l'étape a). Cette alpha-amylase supplémentaire différente de l'Enzyme V peut être choisie parmi les *Bacillus Licheniformis,* notamment celles décrites dans la demande WO 2011/017093 A1. De préférence, les *Bacillus Licheniformis* sont choisies parmi les enzymes de type Spezyme® Alpha (Genencor®), LpHera® (Novozymes®), Liquozyme® Supra (Novozymes®) et Termamyl® 120L (Novozymes®).

Lors des étapes a) et d), le pH des solutions d'amidons peut être augmenté par l'ajout d'au moins une base ou diminué par l'ajout d'au moins un acide dans des quantités suffisantes de manière à sélectionner le pH désiré. L'acide peut être organique ou inorganique et notamment être l'acide chlorhydrique. La base peut être organique ou inorganique et notamment être la soude.

Avantageusement, la solution aqueuse d'amidon formée à l'étape a) présente un pH allant de 4,0 à 6,5, avantageusement de 4,3 à 5,9.

La suspension aqueuse d'amidon peut comprendre d'autres composés additifs aidant à l'hydrolyse enzymatique. Par exemple, elle peut comprendre en outre au moins un sel de calcium, notamment lorsque l'on utilise une alpha-amylase supplémentaire qui est calciodépendante. La suspension aqueuse d'amidon peut également comprendre du bisulfite de sodium.

La solution aqueuse d'amidon peut être préparée par simple mélange des constituants.

Après l'étape de mise en contact, le procédé comprend une étape d'hydrolyse b) pour former une solution d'hydrolysat d'amidon, appelé « hydrolysat intermédiaire ».

Comme indiqué précédemment, la durée et la température de l'étape d'hydrolyse b) dépend directement du pH et de la quantité d'Enzyme V utilisée lors de l'étape a), ainsi que du DE de l'hydrolysat intermédiaire DE, visé.

Cette étape d'hydrolyse b) peut être réalisée en chauffant la solution d'amidon préparée à l'étape a). De préférence, la solution d'amidon est chauffée à une température allant de 75 à 120°C. Cette étape b) est réalisée jusqu'à ce que l'on hydrolyse suffisamment l'amidon, avant de réaliser l'étape d'inhibition c), pour ensuite pouvoir obtenir l'hydrolysat intermédiaire utile à l'invention. La durée de cette étape b) peut aller de 2 à 300 minutes. Cette étape peut se faire dans tout type de réacteur classiquement utilisé pour l'hydrolyse de l'amidon et de préférence agité.

Selon une première variante, cette première étape d'hydrolyse b) peut consister en un stade d'hydrolyse dit « de haute température », la température utilisée lors de ce stade pouvant être comprise entre 100 et 115°C, notamment en utilisant un cuiseur à injection de vapeur, couramment appelé *jet cooker.* Avantageusement, on réalise ce stade de manière à gélatiniser l'amidon ou de rendre l'amidon au moins partiellement soluble. Ce stade peut notamment durer de 2 à 30 minutes, généralement de 3 à 15 minutes. Il peut se faire à une pression d'au moins 1,2 bar, par exemple à une pression allant de 1,3 bar à 8 bar.

Selon une seconde variante, cette première étape d'hydrolyse b) peut comprendre un premier stade d'hydrolyse dit « de haute température » et un second stade d'hydrolyse dit « de basse température », la température utilisée lors du premier stade étant supérieure à celle utilisée lors du second stade. Le stade d'hydrolyse de haute température peut être réalisé comme décrit dans la variante précédente. Le stade d'hydrolyse de basse température peut être réalisé dans tout type de réacteur classique thermostaté, de préférence agité, avantageusement à une température comprise entre 80 et 100°C. Ce second stade peut notamment durer de 5 à 300 minutes.

Le procédé selon l'invention comprend une étape c) d'inhibition de l'Enzyme V. Cette étape d'inhibition peut être réalisée par chauffage de la solution d'hydrolysat intermédiaire. Préférentiellement, l'étape d'inhibition se fait par chauffage de la solution d'hydrolysat intermédiaire, par exemple à une température supérieure ou égale à 140°C voire supérieure ou égale à 150°C. Un avantage de cette étape d'inhibition par chauffage est qu'elle ne nécessite pas d'ajouts successifs d'acide et de base ; ainsi, l'étape éventuelle ultérieure de déminéralisation de l'hydrolysat est plus aisée. Cette étape d'inhibition peut également être réalisée en diminuant le pH de la solution d'hydrolysat intermédiaire, notamment à un pH inférieur à 3,5, en combinaison avec un chauffage de 90°C ou plus. Avantageusement, le pH est compris entre 2,5 et 3, par exemple est de 2,9 environ. De préférence, l'étape d'inhibition a une durée allant de quelques minutes à quelques heures, par exemple allant de 30 à 120 minutes. Cette étape est essentielle au procédé de l'invention, afin de pouvoir obtenir l'hydrolysat à la distribution glucidique particulière de l'invention. A l'issue de cette étape c) d'inhibition, la solution d'hydrolysat intermédiaire inhibée obtenue à l'étape c) peut présenter un $DE_c$ très légèrement supérieur à celui de l'étape b). On considère généralement que l'enzyme V est inhibée lorsque, en plaçant pendant 3 heures la solution d'hydrolysat intermédiaire inhibée à 90°C et à un pH de 5,2, son DE est inférieur ou égal $DE_c+1$, de préférence inférieur ou égal $DE_c+0,5$, tout préférentiellement égal à $DE_c$.

Selon le procédé spécifique de l'invention, $DE_c$ est inférieur au DE de l'hydrolysat $(DE_e)$ récupéré à l'issue de l'étape f) ultérieure et en dépend donc directement.

A l'issue de cette étape d'inhibition c), le DE de l'hydrolysat intermédiaire peut aller de 3 à 20, par exemple de 4 à 15, notamment de 5 à 11.

Suite à l'étape d'inhibition, on réalise une étape d) de mise en contact de la solution d'hydrolysat intermédiaire obtenue à l'étape c) avec au moins une alpha-amylase additionnelle, différente de l'Enzyme V.

L'alpha-amylase additionnelle peut être choisie parmi les *Bacillus Licheniformis,* notamment celles décrites dans la demande WO 2011/017093 A1. De préférence, les *Bacillus Licheniformis* sont choisies parmi les enzymes de type Spezyme® Alpha (Genencor®), LpHera® (Novozymes®), Liquozyme® Supra (Novozymes®) et Termamyl® (Novozymes®).

Lors de l'étape d), l'alpha-amylase additionnelle est mise en contact dans des quantités permettant d'obtenir l'hydrolysat visé à l'issue de l'étape e). Cette quantité peut varier très largement et est directement liée aux conditions utilisées lors de cette étape e), notamment la durée, la température, et/ou le pH de la suspension aqueuse d'amidon. L'alpha-amylase additionnelle est généralement introduite sous forme de solutions commerciales d'enzymes. Par exemple, on peut l'introduire sous la forme d'une solution commerciale d'enzyme dans des quantités massiques, exprimées par rapport à la masse sèche d'amidon, allant de 0,01 à 0,5%, de préférence de 0,02 à 0,13%. Une autre manière d'exprimer la quantité d'alpha-amylase additionnelle est de l'exprimer en quantité d'activité enzymatique de type «Novo Unit » (NU), bien connue de l'Homme du métier. Par gramme d'amidon sec, cette quantité peut être comprise dans la gamme allant de 10 à 1000 NU, par exemple de 50 à 500 NU. A titre d'exemple, 1 g d'une solution d'enzyme Liquozyme Supra ® comprend au moins 136000 NU et 1 g d'une solution d'enzyme LpHera ® comprend au moins 400000 NU.

Pour éviter toute mauvaise interprétation, il est précisé que, lorsque l'on utilise plusieurs alpha-amylases additionnelles selon le procédé de l'invention, aucune de ces alpha-amylases mise en contact avec l'hydrolysat lors de l'étape d) n'est une Enzyme V. Ainsi, l'étape d'hydrolyse e) est réalisée en absence d'Enzyme V. De préférence, aucune enzyme autre que les alpha-amylases additionnelles ne sont mises en contact avec l'hydrolysat lors des étapes d) et e). Les conditions de pH peuvent être sélectionnées de manière à ce que l'alpha-amylase présente une activité enzymatique lors de l'étape e).

Avantageusement, la solution d'hydrolysat formée à l'étape d) présente un pH allant de 4,3 à 5,9.

La solution d'hydrolysat formée à l'étape d) peut comprendre d'autres composés additifs aidant à l'hydrolyse enzymatique. Par exemple, elle peut comprendre en outre au moins un sel de calcium, notamment lorsque l'on utilise une alpha-amylase additionnelle qui est calciodépendante. La suspension aqueuse d'amidon peut également comprendre du bisulfite de sodium.

La solution d'hydrolysat formée à l'étape d) peut être préparée par simple mélange des constituants.

Pour obtenir le profil particulier de l'hydrolysat selon l'invention, le procédé selon l'invention comprend deux étapes d'hydrolyse enzymatique b) et e), réalisées avec différentes alpha-amylases. Ainsi, l'étape e) d'hydrolyse enzymatique est conduite de manière à ce que le Dextrose Equivalent $DE_e$ soit supérieur au Dextrose Equivalent $DE_c$ de l'hydrolysat intermédiaire, c'est-à-dire que l'étape e) est réalisée dans des conditions de temps et de température permettant d'augmenter le Dextrose Equivalent de l'hydrolysat.

Avantageusement, le Dextrose Equivalent $DE_e$ est supérieur ou égal à $DE_c$ + 1, de préférence supérieur ou égal à $DE_c$ + 3, notamment supérieur ou égal à $DE_c$ + 5, par exemple supérieur ou égal à $DE_c$ + 7. Evidemment, le profil glucidique de l'hydrolysat varie, dans les bornes décrites précédemment, et celui-ci dépend de la différence entre les Dextroses Equivalents $DE_c$ et $DE_e$. Plus cette différence sera importante, plus le profil glucidique se différencie du profil d'un hydrolysat obtenu par une étape d'hydrolyse enzymatique utilisant exclusivement l'Enzyme V lors de l'étape d'hydrolyse.

Cette étape d'hydrolyse e) peut être réalisée en chauffant l'hydrolysat intermédiaire obtenu à l'étape c). Les conditions de température et de temps peuvent varier largement selon l'hydrolysat visé. Elles peuvent être sélectionnées, en fonction du pH et des quantités d'enzymes additionnelles utilisées, de manière à obtenir le Dextrose Equivalent $DE_e$ souhaité.

De préférence, ledit hydrolysat intermédiaire est chauffé à une température allant de 50 à 120°C. Cette étape e) est réalisée jusqu'à ce que l'on obtienne cet hydrolysat intermédiaire. La durée de cette étape e) peut aller de 10 à 1500 minutes.

Le procédé selon l'invention comprend une étape f) de récupération de l'hydrolysat formé à l'étape e) précédente.

Cette étape peut comprendre en outre une étape ultérieure g) de transformation de l'hydrolysat récupéré à l'étape f).

L'étape de transformation g) peut consister une étape de purification de l'hydrolysat. Cette étape peut notamment être une étape de filtration, par exemple par passage sur filtre-presse ou sur une membrane de microfiltration ou d'ultrafiltration. Il peut également s'agir d'une étape de déminéralisation de l'hydrolysat, par exemple par passage sur résine échangeuse d'ions. Il peut également s'agir d'une étape de décoloration de l'hydrolysat, par exemple en mettant en contact l'hydrolysat avec des charbons actifs.

L'étape de transformation peut également consister en une étape de concentration par évaporation de l'eau de l'hydrolysat ou encore en une étape de dilution de l'hydrolysat avec de l'eau.

A l'issue de l'étape de récupération f) ou à l'issue des étapes de traitement précédemment citées, l'hydrolysat obtenu se présente sous forme d'une solution aqueuse.

Il est divulgué également un hydrolysat d'amidon qui se présente sous forme de solution aqueuse, dont la quantité massique d'hydrolysat, exprimé en masse sèche, va avantageusement de 20 à 85% et va préférentiellement de 50 et 80%.

Le procédé de l'invention peut également comprendre différentes étapes de transformations parmi celles décrites précédemment.

Le procédé selon l'invention peut également comprendre une étape de transformation g) qui est une étape de mise en forme de l'hydrolysat sous forme de poudre, notamment par une étape d'atomisation. Dans le cas où on réalise différentes étapes de transformation, cette étape d'atomisation peut être l'étape de transformation finale.

L'hydrolysat peut ainsi se présenter sous forme d'une poudre, pouvant présenter une quantité massique en hydrolysat, exprimée en masse sèche, supérieure à 90%. L'invention va maintenant être décrite en détail dans les exemples ci-après. L'Homme du métier saura aisément, à la lecture de la description de la demande, adapter les conditions du procédé afin d'obtenir les hydrolysats.

## Exemples

## Fabrication et caractérisation des hydrolysats

Enzymes utilisées

*Enzyme V:* Enzyme *Veretase® (Verenium®)*.
Autres alpha-amylases :

*LpHera® (Novozymes®)* ;

*Liquozyme® Supra (Novozymes®)* ;

*Termamyl® 120L (Novozymes®)*.

Méthodes

*Chromatographie Liquide Haute Performance*

Le système de chromatographie liquide haute performance est composé d'une pompe de type WATERS M515, d'un injecteur automatique de type WATERS WISP, d'un four de thermostatisation de colonnes réglé à 55°C, d'un réfractomètre différentiel de type WATERS R2414 et d'un système informatique équipé d'un logiciel permettant de traiter les chromatogrammes de type Empower (WATERS). Deux colonnes de résine échangeuse d'ions sous forme argent de type Aminex HPX - 42A Carbohydrate Column (300 mm $\times$ 7,8 mm) montées en série sont utilisées. L'éluant utilisé est de l'eau distillée (débit : 0,4 ml/minute). Un échantillon de la solution d'hydrolysat à analyser est préparé en diluant avec de l'eau distillée ladite solution à 5% de matière sèche environ, puis en la filtrant en la passant sur une seringue équipé d'un embout composé d'une membrane filtrante (porosité 0,45 $\mu$m). 20 $\mu$L de cette solution sont ensuite injectés dans l'appareil pour analyse.

*Chromatographie d'exclusion stérique*

Le système de chromatographie d'exclusion stérique est un chromatographe liquide haute performance composé d'une pompe haute pression de type WATERS 510-type, d'un injecteur WATERS 717+, d'un four de thermostatisation de colonnes réglé à 35°C, d'un réfractomètre différentiel de type WATERS R2414 et d'un système informatique équipé d'un logiciel permettant de traiter les chromatogrammes de type Empower (WATERS) équipé de l'option SEC. Trois colonnes sont connectées en série et placées dans l'ordre suivant : OHpak SB-802 HQ, OHpak SB-803 HQ, OHpak SB-805 HQ (Waters). L'éluant utilisé (0,5 ml/minute) est une solution aqueuse réalisée à partir d'eau distillée, de nitrate de sodium (concentration dans la solution aqueuse de 0,1M) et d'azoture de sodium (concentration à 0,02% en masse). Le chromatographe d'exclusion stérique est calibré à l'aide de standard pullulans. Un échantillon de la solution d'hydrolysat à analyser est préparé en diluant à l'aide de l'éluant ladite solution à 5% de matière sèche environ, puis en la filtrant en la passant sur une seringue équipé d'un embout composé d'une membrane filtrante (porosité 0,45 $\mu$m). 100 $\mu$L de cette solution sont ensuite injectés dans l'appareil pour analyse.

*Dextrose Equivalent*

Le DE est mesuré en utilisant la méthode NF EN ISO 5377:1981.

Procédé de fabrication des hydrolysats

Différents hydrolysats d'amidon comparatifs ont été réalisés selon le protocole 1 suivant :
Dans une cuve est préparée sous agitation active une solution aqueuse d'amidon à 18 - 19 degré baumé (soit 33% MS environ) à partir d'amidon de maïs natif sec et d'eau déminéralisée. Toujours sous agitation, le pH est rectifié (à l'aide d'une solution de HCl ou de NaOH à 0,1 N) pour régler le pH à 5,3, ou à 4,8 dans le cas où l'alpha-amylase utilisée ensuite est l'enzyme LpHera.
Toujours sous agitation, la solution commerciale d'alpha-amylase (CP1 à CP4) ou le mélange de solutions commerciales d'alpha-amylase (CP5 à CP6) est mise en contact avec la solution aqueuse d'amidon dans les proportions massiques par rapport au poids sec d'amidon indiquées dans le Tableau 1. Dans le cas où l'enzyme Termamyl 120L, qui est calcio-dépendante, est utilisée, du CaCl2 est préalablement ajouté à la suspension (60 ppm de calcium environ dans la solution d'amidon).

L'étape d'hydrolyse est réalisée de la manière suivante : la solution d'amidon est introduite dans un cuiseur à injection de vapeur *(jet cooker)*. De la vapeur est injectée dans la solution d'amidon via une tuyère pour atteindre 106°C avec une contrepression à 1,4 bars. La solution d'amidon après éclatement est maintenue à température dans une zone de contact pendant 5 à 6 minutes. L'amidon est solubilisé lors de cette étape et est récupéré à la sortie du cuiseur à injection de vapeur et directement introduit dans un bain marie pour la poursuite de l'hydrolyse. La température est réglée à 95°C. Le DE de l'hydrolysat augmente au fur et à mesure de l'hydrolyse. On récupère différents hydrolysats d'amidon au cours du temps. Après récupération, l'enzyme est immédiatement inhibée en abaissant le pH à 2,9 à l'aide d'une solution d'HCl (0,1 N) et en maintenant l'hydrolysat à 95°C pendant 1h.

Différents hydrolysats d'amidon selon l'invention ont été réalisés selon le protocole 2 suivant :
Dans une cuve est préparée sous agitation active une solution aqueuse d'amidon à 18 - 19 degré baumé (soit 33% MS environ) à partir d'amidon de maïs natif sec et d'eau déminéralisée. Toujours sous agitation, le pH est rectifié (à l'aide d'une solution de HCl ou de NaOH à 0,1 N) pour régler le pH à 5,3.

Toujours sous agitation, la solution commerciale d'Enzyme V est mise en contact avec la solution aqueuse d'amidon, dans des quantités massiques de 0,08% en masse par rapport au poids sec d'amidon.

L'étape d'hydrolyse est réalisée de la manière suivante : la solution d'amidon est introduite dans un cuiseur à injection de vapeur *(jet cooker)*. De la vapeur est injectée dans la solution d'amidon via une tuyère pour atteindre 106°C avec une contrepression à 1,4 bars. La solution d'amidon après éclatement est maintenue à température dans une zone de contact pendant 5 à 6 minutes. L'amidon est solubilisé lors de cette étape et est récupéré à la sortie du cuiseur à injection de vapeur et directement introduit dans un bain marie. L'enzyme V est inhibée immédiatement en abaissant le pH à 2,9 à l'aide d'une solution d'HCl (0,1 N) et en maintenant l'hydrolysat à 95°C pendant 1h. Deux hydrolysats intermédiaires ont été réalisés selon ce protocole ; le premier hydrolysat intermédiaire présente un DE égal à 7 (pour un temps de contact d'environ 6 minutes) et le second hydrolysat intermédiaire de DE égal à 5 (pour un temps de contact d'environ 5 minutes).

Toujours sous agitation, le pH est rectifié (à l'aide d'une solution de NaOH à 0,1 N) pour régler le pH à 5,3 dans le cas où l'alpha-amylase additionnelle ensuite utilisée est l'enzyme Liquozyme Supra ou à 4,8 dans le cas où l'alpha-amylase ensuite utilisée est l'enzyme LpHera.

Selon un premier exemple selon l'invention (INV1), la solution commerciale de Liquozyme Supra est mise en contact avec la solution du premier hydrolysat intermédiaire (DE=7), dans des quantités massiques de 0,1% en masse par rapport au poids sec d'amidon. L'hydrolysat est maintenu à 95°C et le DE de l'hydrolysat augmente au fur et à mesure de l'hydrolyse. On récupère différents hydrolysats d'amidon au cours du temps. Après récupération d'un hydrolysat, l'enzyme est immédiatement inhibée en abaissant le pH à 2,9 à l'aide d'une solution d'HCl (0,1 N) et en maintenant l'hydrolysat à 95°C pendant 1 h.

Selon un second exemple selon l'invention (INV2), la solution commerciale de LpHera est mise en contact avec la solution du second hydrolysat intermédiaire (DE=5), dans des quantités massiques de 0,03% en masse par rapport au poids sec d'amidon. L'hydrolysat est maintenu à 95°C et le DE de l'hydrolysat augmente au fur et à mesure de l'hydrolyse. On récupère différents hydrolysats d'amidon au cours du temps. Après récupération d'un hydrolysat, l'enzyme est immédiatement inhibée en abaissant le pH à 2,9 à l'aide d'une solution d'HCl (0,1 N) et en maintenant l'hydrolysat à 95°C pendant 1 h.

Caractéristiques des hydrolysats

Les teneurs en poids sec des saccharides et les DE des hydrolysats comparatifs et selon l'invention sont analysés selon les méthodes décrites ci-dessus et sont reportés dans les Tableaux 1 et 2 ci-dessous.

Si ces teneurs de DP10-20 ou DP50+ sont conformes aux critères de l'invention, le chiffre indiqué dans la case correspondante est indiqué en gras.

Tableau 1 : DE et profils glucidiques des hydrolysats obtenus par le protocole 1 (comparatifs)

| N° | CP1 | | | | | CP 2 | | | | CP3 | | | | | CP4 | | | | |
|---|---|---|---|---|---|---|---|---|---|---|---|---|---|---|---|---|---|---|---|
| Enzyme | Termamyl 120L (0,1%) | | | | | Liquozyme Supra (0,1%) | | | | Veretase (0,08%) | | | | | LpHera (0,03%) | | | | |
| DE | 12 | 17 | 19 | 21 | 28 | 16 | 19,2 | 22,7 | 25,8 | 13,9 | 18,7 | 20,1 | 22 | 29,3 | 12,1 | 17,5 | 20,6 | 23,7 | 28,1 |
| DP1-2 | 3,8 | 5,7 | 6,5 | 7,6 | 11,2 | 4,2 | 6,1 | 9,2 | 9,9 | 3,9 | 5,5 | 6,7 | 7,7 | 12,6 | 2,3 | 4,6 | 6,7 | 9,1 | 12,9 |
| DP3-6 | 16,5 | 26,2 | 27,4 | 32,1 | 53,3 | 21,2 | 30,2 | 38,1 | 39,8 | 13,0 | 17,5 | 20,7 | 23,5 | 34,3 | 16,3 | 26,8 | 32,5 | 37,7 | 45,0 |
| DP7-9 | 11,9 | 14,2 | 13,8 | 13,7 | 5,7 | 17,2 | 18,3 | 14,9 | 13,6 | 19,4 | 24,6 | 26,9 | 28,3 | 29,1 | 16,7 | 19,6 | 18,2 | 15,4 | 9,5 |
| DP11+ | ND | ND | ND | ND | ND | 53,4 | 42,7 | 35,9 | 34,9 | 54,7 | 42,1 | 36,1 | 30,8 | 15,7 | 61,6 | 47,3 | 41 | 36,5 | 30,5 |
| DP10-20 | 22,4 | 19,6 | 18,4 | 16,7 | 10,8 | 14 | 9,1 | 8,8 | 9,1 | 41,6 | 40,8 | 37,5 | 34,6 | 22,5 | 10,7 | 6,5 | 6,5 | 7,5 | 9,5 |
| -0,83 × DE + 27,5 | 17,5 | 13,3 | 11,7 | 10,1 | 4,3 | 14,2 | 11,6 | 8,7 | 6,1 | 16,0 | 12,0 | 10,8 | 9,2 | 3,2 | 17,5 | 13,0 | 10,4 | 7,8 | 4,2 |
| -1,25 × DE + 55 | 40,0 | 33,8 | 31,3 | 28,8 | 20,0 | 35,0 | 31,0 | 26,6 | 22,8 | 37,6 | 31,6 | 29,9 | 27,5 | 18,4 | 39,9 | 33,1 | 29,3 | 25,4 | 19,9 |
| DP21-30 | 6,9 | 5,8 | 5,5 | 4,8 | 3,3 | 4,5 | 3,7 | 3,8 | 3,9 | 10,1 | 6,9 | 5,4 | 4,1 | 1,2 | 3,4 | 2,7 | 3,1 | 3,6 | 4 |
| DP31-40 | 3,7 | 3,5 | 3,5 | 3,7 | 2,5 | 3,1 | 3 | 3,3 | 3,2 | 4,8 | 2,5 | 1,8 | 1,2 | 0,2 | 2,1 | 2,6 | 2,8 | 3 | 3 |
| DP41-50 | 2,9 | 2,8 | 2,6 | 2,5 | 2,0 | 2,7 | 3 | 2,8 | 2,7 | 2,3 | 0,9 | 0,6 | 0,3 | 0 | 1,6 | 2,5 | 2,7 | 2,7 | 2,4 |
| DP50+ | 31,8 | 22,2 | 22,4 | 18,8 | 11,1 | 33,3 | 26,6 | 19,2 | 18 | 5,1 | 1,3 | 0,7 | 0,4 | 0 | 46,7 | 34,8 | 27,4 | 21,1 | 13,6 |
| -0,83 × DE + 25 | 15,0 | 10,9 | 9,2 | 7,6 | 1,8 | 11,7 | 9,1 | 6,2 | 3,6 | 13,5 | 9,5 | 8,3 | 6,7 | 0,7 | 15,0 | 10,5 | 7,9 | 5,3 | 1,7 |
| -1,07 × DE + 40 | 27,2 | 21,8 | 19,7 | 17,5 | 10,0 | 22,9 | 19,5 | 15,7 | 12,4 | 25,1 | 20,0 | 18,5 | 16,5 | 8,6 | 27,1 | 21,3 | 18,0 | 14,6 | 9,9 |

Tableau 2 : DE et profils glucidiques des hydrolysats obtenus par le protocole 1 avec des mélanges d'enzymes (comparatifs) et le protocole 2 (selon l'invention)

| ° | CP5 | | | | INV1 | | | | CP6 | | | | INV2 | | | | |
|---|---|---|---|---|---|---|---|---|---|---|---|---|---|---|---|---|---|
| Enzyme | 0,08% Liquozyme Supra + 0,02% Veretase | | | | Veretase / inhibition / Licuozyme Supra | | | | 0,05% Liquozyme Supra + 0,05% Veretase | | | | Veretase / inhibition / LpHera | | | | |
| DE | 12,4 | 20,2 | 22,8 | 26,1 | 16,9 | 19,8 | 22,4 | 27,4 | 15 | 18,2 | 22,3 | 28,7 | 12,7 | 20,6 | 21,9 | 24,7 | 28,1 |
| DP1-2 | 3,0 | 6,2 | 7,6 | 10,4 | 3,6 | 5,9 | 7,1 | 11,5 | 3,5 | 5,0 | 7,1 | 12,5 | 2,5 | 5,4 | 6,9 | 10,1 | 11,0 |
| DP3-6 | 11,9 | 28,8 | 31,6 | 39,2 | 22,8 | 31,7 | 36,7 | 49,8 | 13,4 | 18,3 | 25,1 | 39,9 | 16,3 | 29,4 | 35,7 | 42,2 | 46,4 |
| DP7-9 | 16,7 | 24,6 | 25,6 | 24,6 | 21,9 | 23,1 | 21,9 | 12,7 | 18,8 | 23,3 | 27,7 | 26,4 | 18,8 | 24,3 | 23,1 | 18,3 | 14,9 |
| DP11 + | 61,3 | 33,9 | 27,6 | 19,0 | 45,7 | 34,9 | 30,4 | 22,4 | 55,8 | 44,8 | 31,0 | 13,6 | 56,3 | 36,6 | 30,7 | 25,5 | 23,9 |
| DP10-20 | 35,4 | 24,1 | 24,9 | 20,8 | 21,5 | 16,2 | 13,9 | 12,5 | 39,5 | 38,0 | 32,4 | 19,8 | 23,8 | 15,2 | 12,7 | 12,9 | 13,1 |
| -0,83 × DE + 27,5 | 17,2 | 10,7 | 8,6 | 5,8 | 13,5 | 11,1 | 8,9 | 4,8 | 15,1 | 12,4 | 9,0 | 3,7 | 17,0 | 10,4 | 9,3 | 7,0 | 4,2 |
| -1,25 × DE + 55 | 39,5 | 29,8 | 26,5 | 22,4 | 33,9 | 30,3 | 27,0 | 20,8 | 36,3 | 32,3 | 27,1 | 19,1 | 39,1 | 29,3 | 27,6 | 24,1 | 19,9 |
| DP21-30 | 10,7 | 6,0 | 4,9 | 2,9 | 6,8 | 5,1 | 4,6 | 3,8 | 10,4 | 7,9 | 4,8 | 1,1 | 8,9 | 5,5 | 4,8 | 4,4 | 4,2 |
| DP31-40 | 6,0 | 3,1 | 2,1 | 1,0 | 4,3 | 3,5 | 3,2 | 2,4 | 5,0 | 3,3 | 1,6 | 0,2 | 5,3 | 3,7 | 3,2 | 2,7 | 2,5 |
| DP41-50 | 3,3 | 1,8 | 1,1 | 0,4 | 2,9 | 2,4 | 2,2 | 1,6 | 2,5 | 1,4 | 0,6 | 0,1 | 3,2 | 2,5 | 2,1 | 1,8 | 1,5 |
| DP50+ | 13,0 | 5,5 | 2,4 | 0,8 | 16,3 | 12,1 | 10,5 | 6,0 | 6,8 | 2,9 | 0,9 | 0,0 | 21,2 | 14,1 | 11,5 | 7,6 | 6,6 |
| -0,83 × DE + 25 | 14,7 | 8,2 | 6,1 | 3,3 | 11,0 | 8,6 | 6,4 | 2,3 | 12,6 | 9,9 | 6,5 | 1,2 | 14,5 | 7,9 | 6,8 | 4,5 | 1,7 |
| -1,07 × DE + 40 | 26,7 | 18,4 | 15,6 | 12,1 | 21,9 | 18,8 | 16,0 | 10,7 | 24,0 | 20,5 | 16,1 | 9,3 | 26,4 | 18,0 | 16,6 | 13,6 | 9,9 |

Ces Tableaux démontrent que seuls les hydrolysats de l'invention présentent le profil glucidique particulier et combinent l'ensemble des critères DP10-20 ou DP50+. En particulier, l'hydrolysat fabriqué à partir de l'enzyme Termamyl 120L présente systématiquement des quantités de saccharides de type DP50+ toujours supérieur à celui du critère selon l'invention.

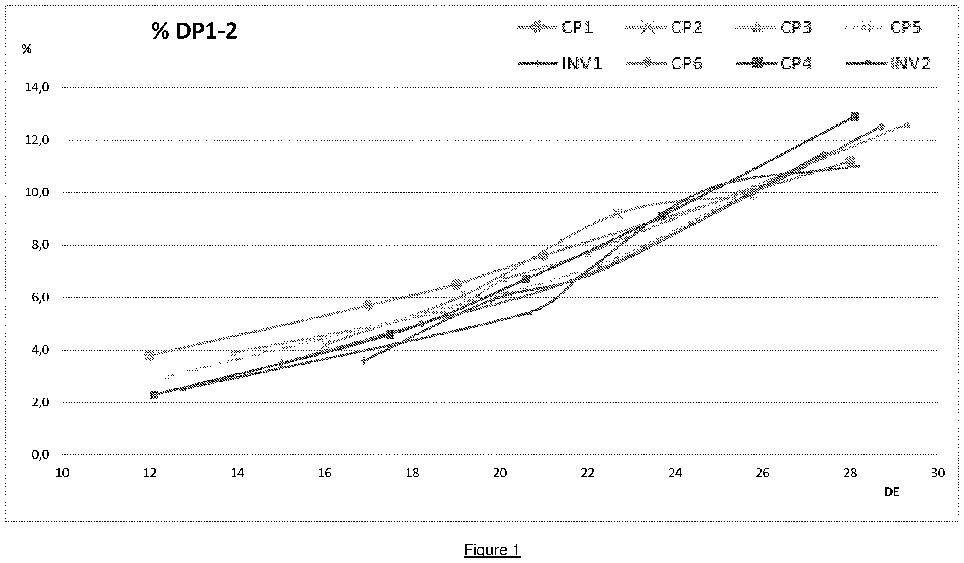

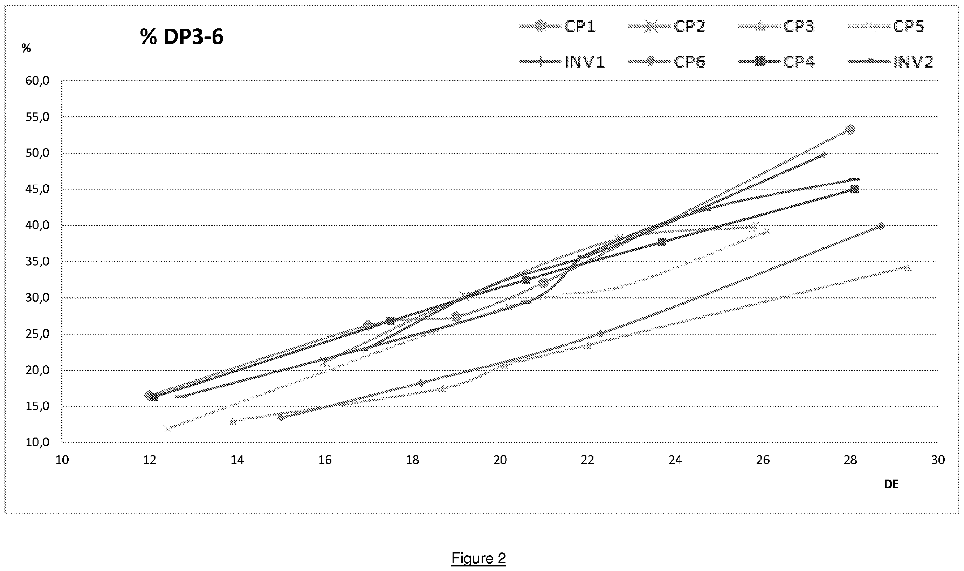

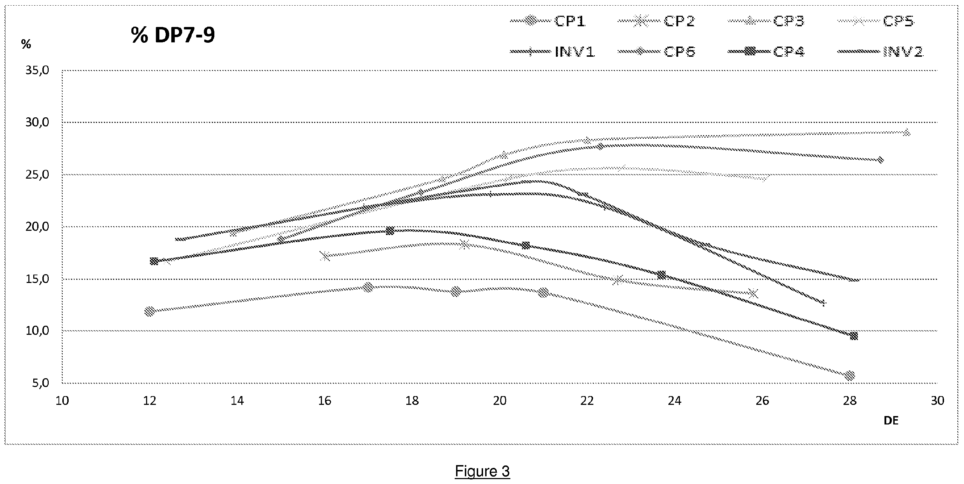

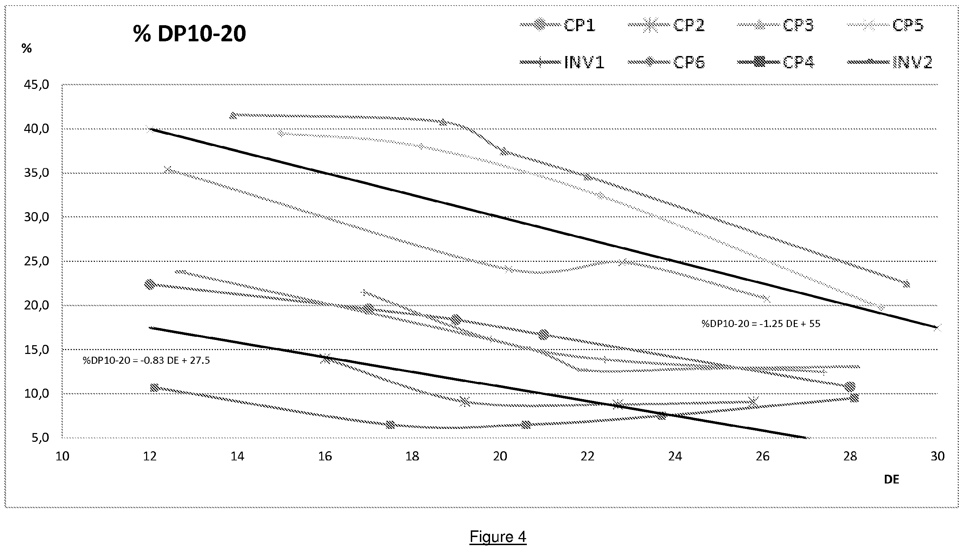

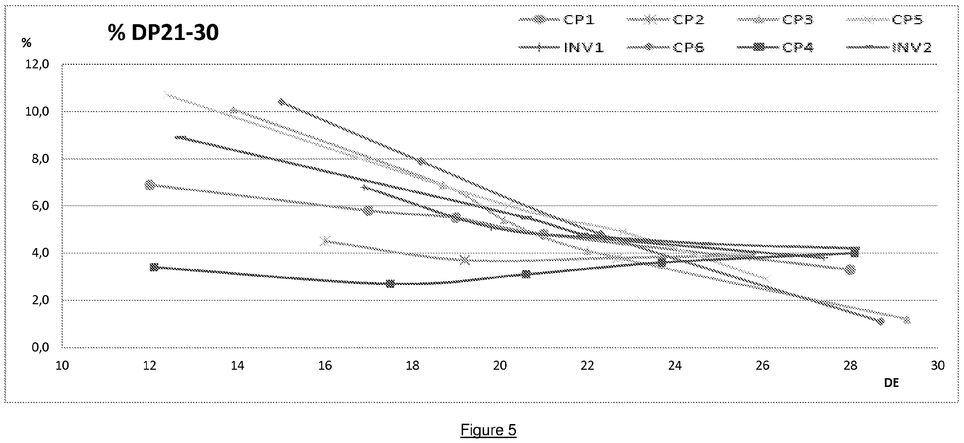

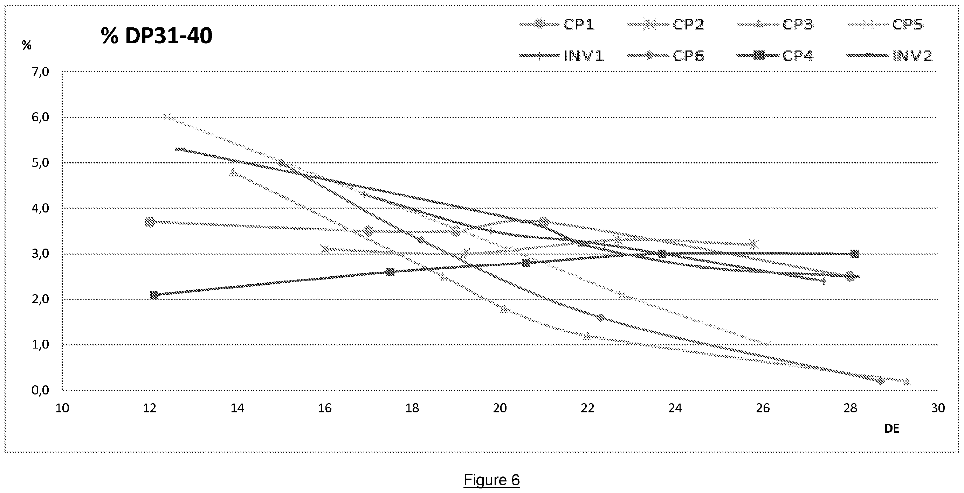

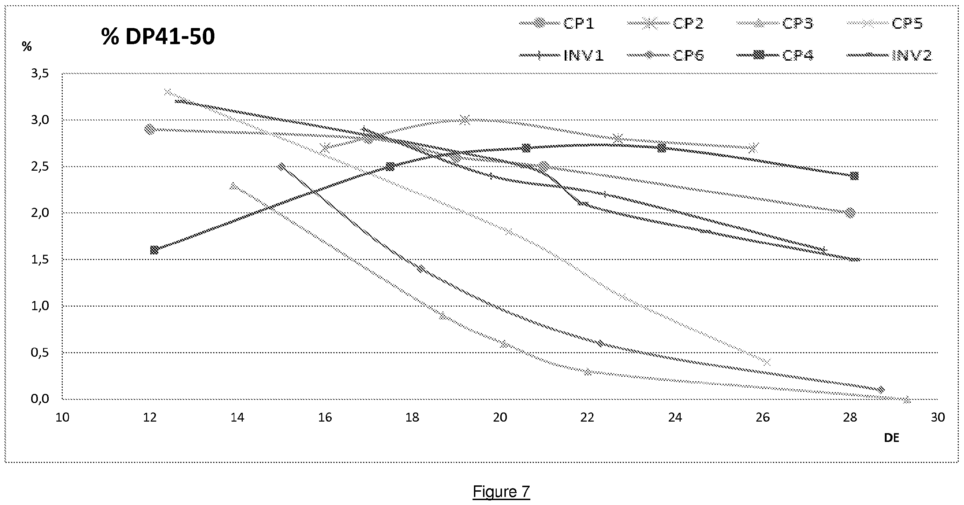

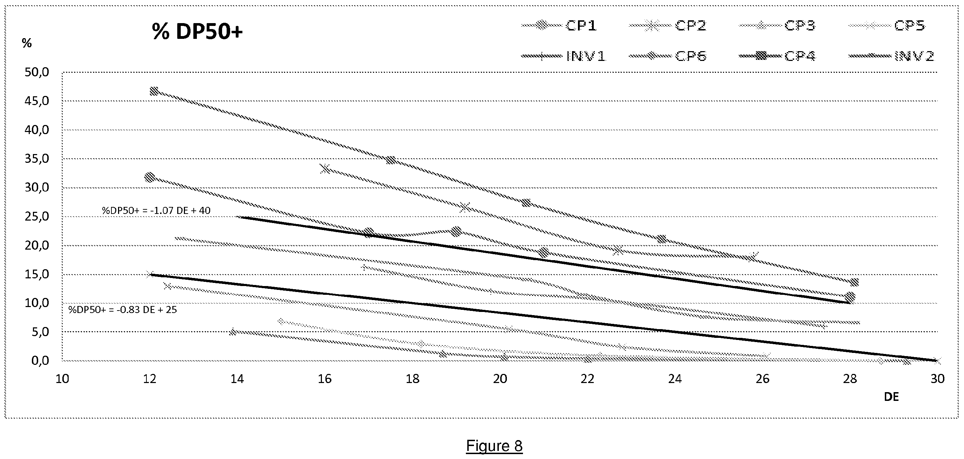

Dans les figures 1 à 8 sont représentées les évolutions des teneurs en poids sec de DPX-Y en fonction du DE des hydrolysats selon l'invention et comparatifs. Les Figures, notamment les Figures 4 et 8, témoignent bien du profil glucidique particulier de l'hydrolysat selon l'invention.

## Test de rétrogradation des hydrolysats d'amidon

Des hydrolysats des essais INV1, CP3 et CP5 sont récupérés et filtrés sous vide en les passant sur un gâteau de terres de diatomées. Ces hydrolysats présentent respectivement un DE de 22,4, 22,0 et 22,8. Ils sont mis sous forme de solutions concentrées à 30% de matière sèche, qui sont alors limpides. Ces solutions sont placées à 4°C pendant 6 heures afin d'accélérer la rétrogradation éventuelle. Après ce traitement, on observe si la solution est rétrogradée ou non, c'est-à-dire si elle est restée limpide ou non. Les observations sont reportées dans le Tableau 3.

Tableau 3 : Comportement à la rétrogradation

| N° hydrolysat | INV 1 | CP3 | CP5 |
|---|---|---|---|
| Aspect de la solution | Limpide | Trouble | Trouble |

Ces essais démontrent que l'hydrolysat selon l'invention présente un comportement à la rétrogradation amélioré. En effet, l'hydrolysat CP3 obtenu à partir de l'Enzyme V et l'hydrolysat CP5 obtenu à partir d'un mélange d'Enzyme V et de *Bacillus Licheniformis* décrit dans la demande WO2011/017093 se troublent lors du test ; au contraire, la solution d'hydrolysat selon l'invention reste toujours limpide au bout de 6 heures.

## Test sensoriel (août et odeur)

On souhaite mesurer l'impact du changement de profil glucidique sur le goût et l'odeur de deux sirops de glucose (DE environ égal à 28).

### Produits

Les deux produits testés sont les hydrolysats INV 1 présentant un DE égal à 27,4 et CP 1 présentant un DE égal à 28.

### Matériel & Méthode

### Mise en oeuvre

Les 2 produits ont été mis en solution pour atteindre un brix final de 27,5. L'eau utilisée pour la mise en solution des produits est déminéralisée avant de l'ajouter au produit.

L'analyse sensorielle a été réalisée par essai triangulaire selon la norme ISO 4120 : 2004.

Pour le test triangulaire d'odeur, les solutions sont présentées aux panélistes dans des flacons en verre fermés maintenus au bain-marie à 60°C.

Pour le test triangulaire de goût, les solutions sont présentées aux panélistes dans des flacons en verre fermés maintenus à température ambiante.

### Panel

Le panel est constitué de participants réguliers à des panels de contrôle qualité concernant les hydrolysats d'amidon.

### Conditions de dégustation

- Box individuels de dégustation, murs blancs, ambiance calme (pour faciliter la concentration)

- Flacons colorés (afin de ne pas être influencé par la vue du produit)

- Produits rendus anonymes par un code à 3 chiffres (pour éviter que le code n'influence l'appréciation des produits)

- Produits présentés dans un ordre aléatoire (pour éviter les effets d'ordre et de rémanence)

Exercice

La méthode employée pour comparer les deux produits est le test triangulaire. Trois échantillons sont présentés simultanément aux dégustateurs, 2 des 3 échantillons sont identiques. L'exercice consiste à identifier quel est l'échantillon différent des 2 autres. Afin d'éviter tout biais, le test a été réalisé en blocs équilibrés (l'échantillon différent est soit l'échantillon A soit l'échantillon B, il se trouve en première, en deuxième ou en troisième position aléatoirement - AAB, ABA, ABB, BAA, BAB, BBA)

Traitement des données

En répondant au hasard à la question « quel est l'échantillon différent? » il y a 1 chance sur 3 de trouver la bonne réponse. Donc, la probabilité d'avoir BR bonnes réponses selon N panélistes sous le fait du hasard suit une loi Binomiale BR~B(N,1/3). Le seuil de confiance usuel est 0.05.

**Résultats**

Odeur

Une différence est bien perceptible entre les produits testés, l'hydrolysat selon l'invention INV1 induit moins de *off flavors* (produit de nettoyage, ciment), il a une odeur plus neutre, moins prononcée.

Goût

Une différence est bien perceptible entre les produits testés. Selon les participants au panel, le produit selon l'invention INV1 est légèrement plus sucré et le produit comparatif présente en outre un léger goût de papier (lié également à la texture de l'hydrolysat), de caramel voire de lessive.

Cet essai démontre, qu'en comparaison avec un hydrolysat de même DE et fabriqué avec une enzyme classique de type Termamyl 120 L, l'hydrolysat présente un goût plus sucré. Ceci est d'autant plus surprenant que les quantités de sucres (DP1-2) des hydrolysats sont, quant à elles, tout à fait semblables à celles des hydrolysats comparatifs CP1.

**Revendications**

1. Procédé de fabrication d'un hydrolysat d'amidon présentant un Dextrose Equivalent DEe allant de 5 à 30 comprenant :

    a) une étape de mise en contact d'une solution aqueuse d'amidon ou de matériau amylacé avec au moins une alpha-amylase comprenant une Enzyme V ;
    b) suivie d'une étape d'hydrolyse de ladite solution aqueuse ;
    c) suivie d'une étape d'inhibition de l'Enzyme V pour former une solution d'hydrolysat intermédiaire présentant un DE égal à $DE_c$ ;
    d) suivie d'une étape de mise en contact de la solution d'hydrolysat intermédiaire obtenue à l'étape c) avec au moins une alpha-amylase additionnelle, différente de l'Enzyme V ;
    e) suivie d'une seconde étape d'hydrolyse pour former un hydrolysat pendant un temps suffisant pour qu'il présente un Dextrose Equivalent $DE_e$ allant de 5 à 30 ;
    f) suivie d'une étape de récupération de l'hydrolysat formé à l'étape e) ;
    **caractérisé en ce que** l'Enzyme V est une enzyme choisie parmi :

        • les polypeptides encodés par une séquence nucléotidique telle que représentée en SEQ ID N°1 ou une séquence nucléotidique présentant un pourcentage d'identité par rapport à la séquence SEQ ID N°1 au moins égal à 80%, ou
• les polypeptides comprenant le fragment enzymatiquement actif de la séquence codée par la séquence SEQ ID N°1 ; et

**en ce que** l'alpha-amylase additionnelle différente de l'Enzyme V est une alpha-amylase de Bacillus Licheniformis.

2. Procédé selon la revendication 1 **caractérisé en ce que** la solution aqueuse d'amidon formée à l'étape a) présente un pH allant de 4,3 à 5,9.

3. Procédé selon l'une des revendications 1 ou 2 **caractérisé en ce que** la solution d'hydrolysat formée à l'étape d) présente un pH allant de 4,3 à 5,9.

4. Procédé selon l'une des revendications 1 à 3 **caractérisé en ce que** l'étape d'inhibition se fait par chauffage de la solution d'hydrolysat intermédiaire à une température supérieure ou égale à 150°C.

5. Procédé selon l'une des revendications 1 à 4 **caractérisé en ce que** $DE_e$ est supérieur ou égal à $DE_c + 3$.

6. Procédé selon l'une des revendications 1 à 5 **caractérisé en ce qu'**il comprend en outre au moins une étape g) de transformation de l'hydrolysat récupéré à l'étape f).

7. Procédé selon la revendication 6 **caractérisé en ce que** l'étape g) est une étape de mise en forme de l'hydrolysat sous forme de poudre par atomisation.

8. Procédé selon l'une des revendications 1 à 7, l'hydrolysat d'amidon présentant un Dextrose Equivalent DE allant de 5 à 30 et dans lequel le DE, la teneur en poids sec de saccharides de degré de polymérisation allant de 10 à 20 (DP 10-20) et la teneur en poids sec de saccharides de degré de polymérisation de 50 ou plus (DP 50+) sont telles qu'ils répondent aux inéquations suivantes :

   •

$$-0,83 \times DE + 25 \leqslant \%DP50+ \leqslant -1,07 \times DE + 40 \ ;$$

   •

$$-0,83 \times DE + 27,5 \leqslant \%DP\ 10\text{-}20 \leqslant -1,25 \times DE + 55 \ .$$

9. Procédé selon la revendication 8, l'hydrolysat d'amidon présentant :

   • un DE allant de 24 à 30 ;
   • une teneur en poids sec de DP 10-20 allant de 10 à 18% ;
   • une teneur en poids sec de DP 50+ allant de 3 à 13%.

10. Procédé selon la revendication 8, l'hydrolysat d'amidon présentant :

   • un DE allant de 20 à 24 ;
   • une teneur en poids sec de DP 10-20 allant de 10 à 20% ;
   • une teneur en poids sec de DP 50+ allant de 6 à 18%.

11. Procédé selon la revendication 8, l'hydrolysat d'amidon présentant :

   • un DE allant de 17 à 20 ;
   • une teneur en poids sec de DP 10-20 allant de 12 à 25% ;
   • une teneur en poids sec de DP 50+ allant de 10 à 19%.

12. Procédé selon la revendication 8, l'hydrolysat d'amidon présentant :

• un DE allant de 15 à 17 ;
• une teneur en poids sec de DP 10-20 allant de 16 à 27% ;
• une teneur en poids sec de DP 50+ allant de 13 à 22%.

**13.** Procédé selon la revendication 8, l'hydrolysat d'amidon présentant :

• un DE allant de 13 à 15 ;
• une teneur en poids sec de DP 10-20 allant de 17 à 30% ;
• une teneur en poids sec de DP 50+ allant de 17 à 25%.

**14.** Procédé selon la revendication 8, l'hydrolysat d'amidon présentant :

• un DE allant de 10 à 13 ;
• une teneur en poids sec de DP 10-20 allant de 20 à 32% ;
• une teneur en poids sec de DP 50+ allant de 18 à 28%.

**Patentansprüche**

1. Verfahren zur Herstellung eines Stärkehydrolysats, das ein Dextrose-Äquivalent DE im Bereich von 5 bis 30 aufweist, umfassend:

a) einen Schritt des Inkontaktbringens einer wässrigen Lösung von Stärke oder stärkehaltigem Material mit mindestens einer alpha-Amylase, die ein Enzym V umfasst;
b) gefolgt von einem Schritt der Hydrolyse der wässrigen Lösung;
c) gefolgt von einem Schritt der Hemmung von Enzym V, um eine intermediäre Hydrolysat-Lösung mit einem DE gleich $DE_c$ zu bilden;
d) gefolgt von einem Schritt des Inkontaktbringens der in Schritt c) erhaltenen intermediären Hydrolysat-Lösung mit mindestens einer zusätzlichen alpha-Amylase, die sich von dem Enzym V unterscheidet;
e) gefolgt von einem zweiten Hydrolyseschritt zur Bildung eines Hydrolysats über einen Zeitraum, der ausreicht, damit es ein Dextrose-Äquivalent $DE_e$ im Bereich von 5 bis 30 aufweist;
f) gefolgt von einem Schritt der Gewinnung des in Schritt e) gebildeten Hydrolysats;
**dadurch gekennzeichnet, dass** das Enzym V ein Enzym ist, das ausgewählt ist aus:

• Polypeptiden, die von einer Nukleotidsequenz kodiert werden, wie sie in SEQ ID Nr. 1 dargestellt ist, oder einer Nukleotidsequenz, die eine prozentuale Identität mit der Sequenz SEQ ID Nr. 1 von mindestens 80 % aufweist, oder
• Polypeptiden, die das enzymatisch aktive Fragment der durch die Sequenz SEQ ID NO: 1 kodierten Sequenz umfassen; und

dadurch, dass die zusätzliche alpha-Amylase, die sich von dem Enzym V unterscheidet, eine alpha-Amylase aus *Bacillus Licheniformis* ist.

2. Verfahren nach Anspruch 1, **dadurch gekennzeichnet, dass** die in Schritt a) gebildete wässrige Stärkelösung einen pH-Wert im Bereich von 4,3 bis 5,9 aufweist.

3. Verfahren nach einem der Ansprüche 1 oder 2, **dadurch gekennzeichnet, dass** die in Schritt d) gebildete Hydrolysat-Lösung einen pH-Wert im Bereich von 4,3 bis 5,9 aufweist.

4. Verfahren nach einem der Ansprüche 1 bis 3, **dadurch gekennzeichnet, dass** der Schritt der Hemmung durch Erhitzen der intermediären Hydrolysat-Lösung auf eine Temperatur von 150°C oder höher erfolgt.

5. Verfahren nach einem der Ansprüche 1 bis 4, **dadurch gekennzeichnet, dass** $DE_e$ größer oder gleich $DE_c$ + 3 ist.

6. Verfahren nach einem der Ansprüche 1 bis 5, **dadurch gekennzeichnet, dass** es außerdem mindestens einen Schritt g) zur Weiterverarbeitung des in Schritt f) gewonnenen Hydrolysats umfasst.

7. Verfahren nach Anspruch 6, **dadurch gekennzeichnet, dass** Schritt g) ein Schritt der Formung des Hydrolysats in Pulverform durch Zerstäubung ist.

8. Verfahren nach einem der Ansprüche 1 bis 7, wobei das Stärkehydrolysat ein Dextrose-Äquivalent DE im Bereich von 5 bis 30 aufweist und wobei das DE, der Trockengewichtsgehalt an Sacchariden mit einem Polymerisationsgrad im Bereich von 10 bis 20 (DP 10-20) und der Trockengewichtsgehalt an Sacchariden mit einem Polymerisationsgrad von 50 oder mehr (DP 50+) so sind, dass sie den folgenden Ungleichungen genügen:

- 

$$0{,}83 \times DE + 25 \leq DP50+ \leq -1{,}07 \times DE + 40;$$

- 

$$0{,}83 \times DE + 27{,}5 \leq DP\ 10\text{-}20 \leq -1{,}25 \times DE + 55.$$

9. Verfahren nach Anspruch 8, wobei das Stärkehydrolysat aufweist:

- einen DE-Wert im Bereich von 24 bis 30;
- einen Trockengewichtsgehalt an DP 10-20 im Bereich von 10 bis 18 %;
- einen Trockengewichtsgehalt an DP 50+ im Bereich von 3 bis 13 %.

10. Verfahren nach Anspruch 8, wobei das Stärkehydrolysat aufweist:

- einen DE-Wert im Bereich von 20 bis 24;
- einen Trockengewichtsgehalt an DP 10-20 im Bereich von 10 bis 20 %;
- einen Trockengewichtsgehalt an DP 50+ im Bereich von 6 bis 18 %.

11. Verfahren nach Anspruch 8, wobei das Stärkehydrolysat aufweist:

- einen DE-Wert im Bereich von 17 bis 20;
- einen Trockengewichtsgehalt an DP 10-20 im Bereich von 12 bis 25 %;
- einen Trockengewichtsgehalt an DP 50+ im Bereich von 10 bis 19 %.

12. Verfahren nach Anspruch 8, wobei das Stärkehydrolysat aufweist:

- einen DE-Wert im Bereich von 15 bis 17;
- einen Trockengewichtsgehalt an DP 10-20 im Bereich von 16 bis 27 %;
- einen Trockengewichtsgehalt an DP 50+ im Bereich von 13 bis 22 %.

13. Verfahren nach Anspruch 8, wobei das Stärkehydrolysat aufweist:

- einen DE-Wert im Bereich von 13 bis 15;
- einen Trockengewichtsgehalt an DP 10-20 im Bereich von 17 bis 30 %;
- einen Trockengewichtsgehalt an DP 50+ im Bereich von 17 bis 25 %.

14. Verfahren nach Anspruch 8, wobei das Stärkehydrolysat aufweist:

- einen DE-Wert im Bereich von 10 bis 13;
- einen Trockengewichtsgehalt an DP 10-20 im Bereich von 20 bis 32 %;
- einen Trockengewichtsgehalt an DP 50+ im Bereich von 18 bis 28 %.

**Claims**

1. A method for manufacturing a starch hydrolysate having a Dextrose Equivalent DEe ranging from 5 to 30 comprising:

a) a step of contacting an aqueous solution of starch or starchy material with at least one alpha-amylase comprising an Enzyme V;

b) followed by a step of hydrolysing said aqueous solution;

c) followed by a step of inhibiting the enzyme V to form an intermediate hydrolysate solution having a DE equal to $DE_c$.

d) followed by a step of contacting the intermediate hydrolysate solution obtained in step c) with at least one additional alpha-amylase, different from the Enzyme V;

e) followed by a second hydrolysis step to form a hydrolysate for a sufficient time so that it has a Dextrose Equivalent $DE_e$, ranging from 5 to 30;

f) followed by a step of recovering the hydrolysate formed in step e);

**characterised in that** the Enzyme V is an enzyme selected from:

• the polypeptides encoded by a nucleotide sequence as represented in SEQ ID No: 1 or a nucleotide sequence having a percentage of identity relative to the sequence SEQ ID No: 1 which is at least equal to 80%, or

• polypeptides comprising the enzymatically active fragment of the sequence encoded by the sequence SEQ ID No: 1; and

**in that** the additional alpha-amylase different from the Enzyme V is a Bacillus Licheniformis alpha-amylase.

2. The method according to claim 1 **characterised in that** the aqueous solution of starch formed in step a) has a pH ranging from 4.3 to 5.9.

3. The method according to one of claims 1 or 2 **characterised in that** the hydrolysate solution formed in step d) has a pH ranging from 4.3 to 5.9.

4. The method according to one of claims 1 to 3 **characterised in that** the inhibition step is carried out by heating the intermediate hydrolysate solution to a temperature which is greater than or equal to 150°C.

5. The method according to one of claims 1 to 4 **characterised in that** $DE_e$ is greater than or equal to $DE_c + 3$.

6. The method according to one of claims 1 to 5 **characterised in that** it further comprises at least one step g) of transforming the hydrolysate recovered in step f).

7. The method according to claim 6 **characterised in that** step g) is a step of shaping the hydrolysate into powder form by atomisation.

8. The method according to one of claims 1 to 7, the starch hydrolysate having a Dextrose Equivalent DE ranging from 5 to 30 and wherein the DE, the dry weight content of saccharides with a degree of polymerisation ranging from 10 to 20 (DP 10-20) and the dry weight content of saccharides with a degree of polymerisation of 50 or more (DP 50+) are such that they satisfy the following inequalities:

•

$$-0.83 \times \mathrm{DE} + 25 \leq \%\mathrm{DP50+} \leq -1.07 \times \mathrm{DE} + 40;$$

•

$$-0.83 \times \mathrm{DE} + 27.5 \leq \%\mathrm{DP\ 10\text{-}20} \leq -1.25 \times \mathrm{DE} + 55.$$

9. The method according to claim 8, the starch hydrolysate having:

• a DE ranging from 24 to 30;

• a dry weight content of DP 10-20 ranging from 10 to 18%;

• a dry weight content of DP50+ ranging from 3 to 13%.

**10.** The method according to claim 8, the starch hydrolysate having:

 • a DE ranging from 20 to 24;
 • a dry weight content of DP 10-20 ranging from 10 to 20%;
 • a dry weight content of DP50+ ranging from 6 to 18%.

**11.** The method according to claim 8, the starch hydrolysate having:

 • a DE ranging from 17 to 20;
 • a dry weight content of DP 10-20 ranging from 12 to 25%;
 • a dry weight content of DP50+ ranging from 10 to 19%.

**12.** The method according to claim 8, the starch hydrolysate having:

 • a DE ranging from 15 to 17;
 • a dry weight content of DP 10-20 ranging from 16 to 27%;
 • a dry weight content of DP50+ ranging from 13 to 22%.

**13.** The method according to claim 8, the starch hydrolysate having:

 • a DE ranging from 13 to 15;
 • a dry weight content of DP 10-20 ranging from 17 to 30%;
 • a dry weight content of DP50+ ranging from 17 to 25%.

**14.** The method according to claim 8, the starch hydrolysate having:

 • a DE ranging from 10 to 13;
 • a dry weight content of DP 10-20 ranging from 20 to 32%;
 • a dry weight content of DP50+ ranging from 18 to 28%.

EP 3 387 020 B1

Figure 1

Figure 2

Figure 3

Figure 4

Figure 5

Figure 6

Figure 7

Figure 8

EP 3 387 020 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 7273740 B
- US 7666633 B
- US 7659102 B
- WO 2009137839 A
- WO 2013116175 A
- WO 2011017093 A
- FR 2203877 A1

- GB 2001075 A
- WO 0196537 A2
- EP 0905256 A1
- WO 2004064540 A1
- FR 2762616 A1
- WO 2011017093 A1